# EUROPEAN PATENT SPECIFICATION

(11) **EP 1 406 672 B1**
(45) Date of publication and mention of the grant of the patent: **06.12.2006**
(21) Application number: 02749028.3
(22) Date of filing: 19.07.2002
(51) Int. Cl.: A61L 2/28, A61L 2/24, G01J 1/42, G01N 21/33, G01N 33/487, A61L 2/10, A61L 2/00

(54) **UV IRRADIATION CONTROL**
UV-BESTRAHLUNGSSTEUERUNG
CONTROLE DE L'EXPOSITION AUX RAYONS UV

(30) Priority: 19.07.2001 GB 0117571
(43) Date of publication of application: 14.04.2004
(73) Proprietor: COMMON SERVICES AGENCY, Edinburgh EH5 3SE (GB)
(72) Inventor: LI, Qiangyi, Edinburgh EH9 3EQ (GB); MACLEOD, Alexander, James, Edinburgh EH9 3AY (GB); FOSTER, Peter, Reynolds, Edinburgh EH3 5AN (GB)
(74) Representative: Szczuka, Jan Tymoteusz
(86) International application number: PCT/GB2002/003306
(87) International publication number: WO 2003/007998

(56) References cited:
- WO-A-00/20045

## Description

The present invention relates to the control of UV irradiation of biological fluids and the like.

The irradiation of biological fluids such as blood and blood products, with UV, is an important measure in inactivating more or less dangerous contaminants such as viruses. In order to ensure the safety of the irradiated product it is clearly vital that it should receive a sufficient dosage of the UV radiation. On the other hand such products generally contain important components (e.g. immunoglobulins) which are sensitive to UV radiation and can be more or less readily damaged or degraded by excessive irradiation. There is often only a relatively small margin between a radiation dosage sufficient to achieve an acceptable degree of virus inactivation (expressed as log₁₀ of the reduction in the virus titre or the "log kill") of the contaminating virus(es) and/or other troublesome microorganisms, whilst minimizing the degree of damage to the desired components / active ingredients of the biological fluid. Accordingly it is very important to be able accurately to monitor and control the UV irradiation dosage actually applied to the fluid.

One generally traditional approach has been to use chemical actinometry wherein is used, in place of the biological fluid, a solution containing a chemical reagent which, upon irradiation with UV, undergoes a chemical reaction which produces a physical change such as a change in absorption at a given wavelength, which change is proportional to the incident radiation dosage. It will be appreciated, though, that this somewhat indirect method has practical disadvantages in that it is based upon the assumption that UV irradiation will be identical and constant throughout the period when the biological fluid is irradiated which of course cannot be guaranteed. Thus, if for example, there is a variation in the radiation output of the UV radiation source when the biological fluid is being treated, then the chemical actinometry measurements before and after processing of the biological fluid would not detect this. Another problem that can occur is that the physical change produced by the chemical reaction is non-linear due to consumption of one or more reactants involved in the chemical reaction, and this can result in difficulties in obtaining accurate measurements of the incident UV radiation.

It is an object of the present invention to avoid or minimize one or more of the above disadvantages or problems.

We have now found that UV radiation of biological fluids containing proteinaceous material provides an increase in UV absorbance of the fluid, which increase has a peak at around 314 nm, and which is substantially directly proportional to the amount of UV radiation (dose thereof), absorbed by the biological fluid.

Thus the present invention provides a method of monitoring the dosage of UV-rradiation received by a liquid containing at least one protein selected from albumin, immunoglobulins and fibrinogen; which method comprises the steps of UV-irradiating the liquid; and measuring the increase in absorbance which has a peak in the region from 305 to 325 nm, due to said UV-irradiation; so as to measure the dosage thereof.

The liquid is preferably substantially free of any dye or photosensitive chemical.

In particular embodiments, the fluid (i.e. liquid) also contains at least one micro-organism contaminant; and the step of UV-irradiating the liquid is carried out so that the liquid has received a LTV-irradiation dosage sufficient to achieve a required level of inactivation of said at least one contaminant.

Irradiation can be terminated when the required level of inactivation is achieved.

It will be understood that, in common with other absorption peaks in this part of the electromagnetic radiation spectrum, the absorbance peak monitored in accordance with the present invention does not have a single sharply defined wavelength but extends across a range of frequencies. Thus although the maximum absorbance of this peak is at around 314 nm, the relative increase in absorbance of this peak could be monitored by means of measuring absorbance at closely similar wavelengths i.e. anywhere in the range from 305 to 325 nm.

Thus by means of the present invention it is possible to monitor and control the sterilization of biological fluids by means of UV irradiation thereof, in a particularly simple and precise manner, thereby substantially improving the safety of the treated products whilst minimizing the damage to and degradation of the desirable active components of those fluids. Moreover it is a particular advantage of the present invention that the measurements may be carried out on the biological fluid being irradiated thereby ensuring the most immediate, direct and accurate form of measurement of the radiation dosage actually received. This is particularly significant in the context of irradiation treatments where the fluid being irradiated is passed through an irradiation zone and the radiation dosage actually received is subject to variation as a result of, for example, fluctuations in the fluid flow rate and/or in the radiation output of the UV lamp or other UV radiation source, and/or due to fouling of the passage side walls in the irradiation zone due to build up of deposits thereon. Whilst other, albeit considerably less convenient or accurate techniques may be available for monitoring variations in radiation source output and in flow rate, it is particularly difficult to monitor the effects of fouling during the processing of a biological fluid.

Another particularly significant advantage of the present invention is that it does not require the addition of any dyes or other chemicals (e.g. photosensitive chemicals) to the fluid being irradiated thereby avoiding contamination of the biological fluid with extraneous chemicals and/or the need for further processing in order to remove the chemicals from the biological fluid after the irradiation treatment, before use thereof. Nevertheless it is also possible to monitor.and determine UV radiation dosage by means of incorporating a polypeptide into a fluid being irradiated (whether or not this already contains some proteinaceous material), and measuring the change in absorption of the fluid. Physiologically acceptable and non-pathogenic proteins selected from albumin, immunoglobulins and fibrinogen which can be safely retained in the treated biological fluid without interfering with its intended end use are used.

It is further possible to use a polypeptide-containing radiation dosage detection fluid which is kept separate from the biological fluid, the irradiation of which it is desired to monitor or control, for example, by irradiating the dosage detection fluid in series (i.e. just before or just after in the same locus) or in parallel (i.e. at the same time in a closely adjacent locus) with said biological fluid, although it will be appreciated that this will generally give a less precise measure of the radiation dosage actually received by said biological fluid.

It will be appreciated that the method of the present invention may be used with any kind of UV irradiation fluid treatment system including both batch and continuous flow systems. The method is, however, particularly advantageous when used in combination with continuous flow systems wherein a body of fluid to be treated is passed through a tubular vessel or the like disposed in an irradiation zone since it is particularly difficult to detect temporary fluctuations in irradiation dosage in such systems due to temporary variations in fluid flow rate and/or radiation output from the UV radiation source.

The measurement of the change in UV absorption may be made on an absolute or relative basis. Thus, for example, there may simply be monitored the UV absorption of the fluid (after irradiation thereof) relative to a predetermined absorption value expected (or determined) for the protein content thereof prior to UV irradiation thereof. In general though, the UV absoption of the fluid (after irradiation thereof) will be compared directly with that of the fluid before irradiation thereof. The comparison may be determined with reference to a single determination of UV absorption of a sample of the fluid prior to irradiation thereof, or on a continuous comparison basis by continuously monitoring the difference in absorption for other reasons e.g. due to fluctuation in the concentration of the protein in the fluid, may be minimised. The latter procedure has the further advantage of isolating the absorbance change due to the UV irradiation, from the pre-existing absorbance of the fluid, so that the change may be monitored more readily and/or more precisely.

The method of the present invention can be carried out in an apparatus suitable for use in the UV-irradiation of a biological fluid containing a desired component and a contaminating micro-organism, and which includes at least one proteinaceous component, (and preferably is substantially free of any dye or photosensitive chemical), which apparatus comprises a longitudinally extending vessel having wall means of a UV-transparent material disposable, in use of the apparatus, in close proximity to a UV radiation source within an irradiation area and having an inlet and outlet and a passage means (preferably formed and arranged so as to define a flow path extending therebetween which is substantially free of substantial discontinuities so as to avoid substantially turbulence in fluid flowing therealong in use of the apparatus), and
having an irradiation zone adjacent said UV-transparent wall means for receiving UV radiation from said UV radiation source, in use of the apparatus,
said passage means preferably having a static flow mixing means with a multiplicity of mixer elements for repeatedly subjecting the fluid flow to a mixing operation comprising dividing and re-mixing of the fluid flow, in use of the apparatus, which static flow mixing means extends along said flow path along at least said irradiation zone,
said vessel preferably having an internal diameter of at least 0.1 mm, advantageously at least 1mm, most preferably at least 4 mm, and
said apparatus including fluid flow supply means formed and arranged for passing fluid through said vessel, in use of the apparatus,
so that said fluid flow is preferably subjected to at least 20 said mixing operations,
wherein is provided at least one spectrophotometer device in proximity to a downstream end portion of said irradiation zone, said spectrophotometer device being formed and arranged for measuring, in use of the apparatus, the increase in absorbance of said fluid which has a peak in the region from 305 to 325 nm, due to said UV-irradiation, at at least one wavelength in the range from 305 to 325 nm.

For the avoidance of doubt the term "spectrophotometer" (or alternatively "spectrometer") is used herein to indicate any kind of device capable of monitoring absorbance (absolute or relative) at one or more different wavelengths.

Advantageously the apparatus is formed and arranged so as to monitor the difference in absorbance of the fluid before and after irradiation. This could in principle be done by providing first and second spectrophotometer devices upstream and downstream of the irradiation zone and a comparator coupled thereto so as to obtain the difference in absorbance therebetween. Most conveniently though there is simply used a single spectrophotometer device with the fluid upstream of the irradiation zone being used as the reference fluid in the spectrophotometer device. For the avoidance of doubt, the term spectrophotometer is used herein to indicate any device which can measure absorbance across a wider or narrower range of different wavelengths, or a device which can only measure absorbance at a single wavelength.

It will be appreciated that with such an apparatus, any excursions of the radiation dosage received by the fluid, outside of predetermined acceptable limits, can be readily detected. Advantageously therefore, the apparatus could be provided with alarm means and/or flow control adjustment means operable in response to such excursions so as to alert an operative thereto and/or to automatically adjust the fluid flow rate so as to bring the radiation dosage actually received back within acceptable limits. Thus, for example, if the radiation dosage received falls below a desired limit, the fluid flow rate could be reduced so as to increase the fluid residence time within the irradiation zone, thereby increasing the radiation dosage received, and vice versa.

Accordingly in a preferred apparatus of the invention, the fluid flow supply means is provided with a fluid flow rate control device, and an output of said at least one spectrophotometer, or, where present, said comparator device, is coupled to said fluid flow control device so as to adjust the fluid flow rate in response to excursions of the detected increase in absorbance outside predetermined limits, so as to bring said increase in absorbance back within said predetermined limits. Alternatively or additionally, there is desirably provided an alarm means coupled to an output of said at least one spectrophotometer, or, where present, said comparator device, so as to generate an alarm signal, in use of the apparatus, in response to excursions of the detected increase in absorbance outside predetermined limits.

It will of course be appreciated that whilst we have found that the increase in absorbance of the fluid, following UV irradiation, is substantially directly proportional to the radiation dosage, for a wide range of proteinaceous component concentrations and UV radiation dosages, the absolute increase in absorbance will depend on the concentration of the proteinaceous component(s). It will also be appreciated that monitoring of the increase in absorbance due to UV radiation, can also be applicable to fluids having particularly high absorbance values at the wavelength used to monitor the increase, (whether this be due to high concentrations of proteinaceous material components or other components) with appropriate dilution of the fluids. The actual dilution for which radiation dosage can be monitored will of course depend on the sensitivity of the spectrophotometer device(s) used. We have found though that using more or less readily available devices such as, for example, ATI UNICAM UV/Vis Spectrometer UV2, it is possible to monitor the received radiation dosage of biological fluids typically involved in UV sterilization treatment such as blood fraction products including inter alia 4%(w/v) and 20%(w/v) human albumin and 5% (w/v) human immunoglobulin solutions with relatively high protein concentrations corresponding to those used in practical applications. (In the case of particularly high concentrations with absorbance values of 3 or more the biological fluid may need to be diluted in order to bring the absorbance down to a value which may be more readily measured.) This is particularly useful in relation to monitoring UV radiation dosage in micro-organism inactivation systems such as those disclosed in WO 00/20045 which, unlike other known systems which can only be used with very dilute solutions which then require concentration processes to convert them into a form suitable for practical use, provide highly effective micro-organism inactivation in relatively concentrated biological fluids.

With regard to UV radiation dosage, the measurement of particularly high UV radiation dosages is not particularly useful as such dosages will generally result in unacceptable levels of damage and degradation of useful components in blood fractionation products and other such biological fluids. In practice we have found that UV radiation dosages providing up to a calculated degree of inactivation of canine parvovirus of about 60 logs (measured as >7 logs), are still within the linear range of the increase in absorbance relative to UV radiation dosage. In fact with such very high radiation dosages there will generally be an unacceptably high level of damage to the useful protein components of the biological fluid. This does though, indicate that the linear range of increase of A₃₁₄ extends across the whole of the practically useful range of UVC radiation doses for biological fluid sterilisation.

Further preferred features and advantages of the invention will appear from the following examples and detailed description provided for the purposes of illustration and illustrated with reference to the accompanying drawings in which:
Fig. 1 is a schematic diagram of a an UV sterilization apparatus provided with a radiation monitoring system in accordance with the present invention;
Figs. 2a & b are UV spectrographs of human albumin solutions before and after UV irradiation;
Fig. 3 is a difference spectrograph of an human albumin solution before and after UV irradiation thereof;
Fig. 4 is a graph showing the relation between OD and increasing UV radiation dosage for aqueous human albumin;
Fig. 5 is a similar graph for human albumin spiked with Øx174;
Fig.6 is a graph showing the relation between OD and increasing UV radiation for aqueous IgG at different concentrations;
Fig. 7 is a similar graph for a solution of fibrinogen; and
Fig. 8 is a similar graph at three different concentrations of albumin.

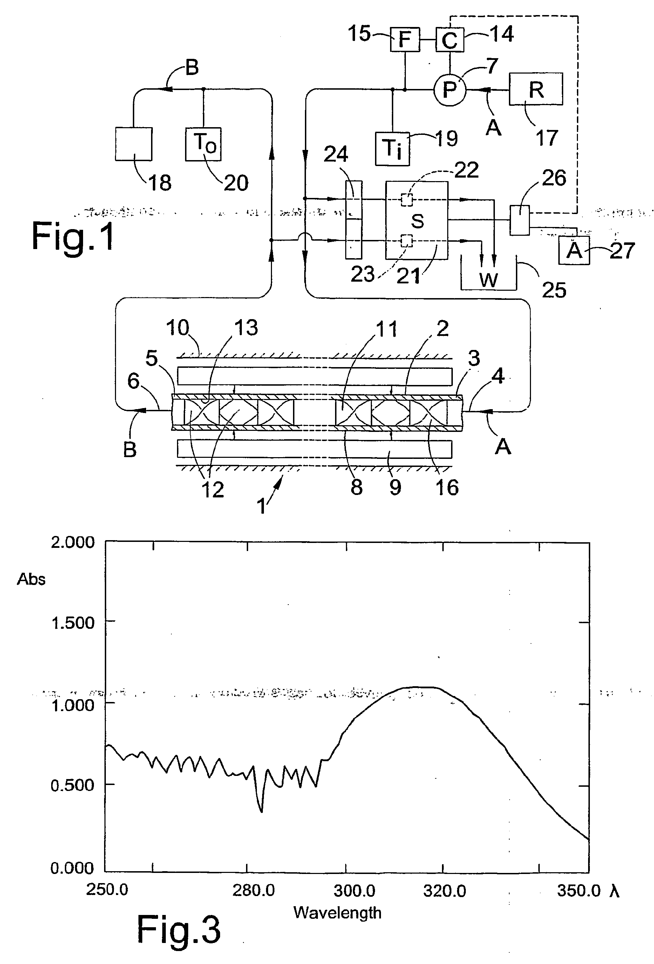

Fig. 1 shows schematically an apparatus 1 of the present invention generally comprising a tubular vessel 2 having a first end 3 with an inlet 4 and a second end 5 having an outlet 6. Arrow A shows the direction of flow of the fluid into the device and arrow B indicates the direction of the flow of the fluid exiting the device in use.

A fluid flow supply means 7 is provided to pass fluid through the tubular vessel 2 in use of the apparatus. The fluid supply means 7 is typically a pump which can pump the fluid through the device at a desired flow-rate, for example, a peristaltic pump or a gear pump.

The tubular vessel 2 of the apparatus 1 is in the form of a cylindrical flourinated ethylene propylene (FEP) tube 8 (alternatively a silica glass tube could be used) and has a length of about 50 cm, an internal diameter 6 mm and a wall thickness of about 1 mm. Four angularly distributed UV-C lamps 9 mounted inside a reflective housing 10 are positioned more or less closely adjacent around the tube 8 with a typical separation of about 5 mm therefrom.

In relation to the control of the exposure of the fluid to the UV radiation, this is monitored substantially directly by continuously monitoring the change in OD₃₁₄ of the fluid 16 between the inlet and outlet ends 4,6 of the tube 8 as described in more detail hereinbelow.

A static flow mixer 11 extends along the length of the vessel 2 and has a series of 80 mixer elements 12 arranged longitudinally thereon with 40 pairs of alternatively handed screw elements angularly offset from each other by 90°. The mixer device used was of Polyamide and had an outside diameter of 6 mm which was a push-fit inside the silica tube vessel 2. The mixer device used was one commercially available from Metermix Systems Ltd of Wellingborough, England under the designation. The elements 12 in such devices are formed and arranged such that in use the fluid is very thoroughly mixed so that different portions of the main body of the fluid are successively brought within a more or less shallow irradiation zone adjacent the wall 8 of the vessel 2 to be UV-irradiated. In this way substantially all of the fluid is exposed to a similar micro-organism inactivating level of UV-irradiation.

In order to control the fluid flow rate through the vessel, the pump 7 is provided with a control means 14 for adjusting the pumping rate. A flow meter 15 of the Coriolis mass flow type, is provided to monitor the volume of fluid passing through the apparatus and may be used to provide a direct input to the pump controller 14 or could simply provide a read out which can be used by the operator, manually to adjust the controller 14. The fluid 16 to be treated is placed initially in a reservoir 17 and after treatment is collected in a sterile container 18.

The temperature of the fluid 16 can be monitored through temperature probes 19, 20 at the inlet and outlet 4,6 of the vessel 2. In practice the temperature rise is generally limited to about 1 to 2°C.

The change in OD₃₁₄ is monitored by means of a spectrophotometer 21 in which the reference cell 22 is connected to a tube carrying a small proportion of the fluid flow which is drawn off continuously from the fluid flow A into the tubular vessel 2, and the sample cell 23 is connected to a tube carrying fluid which is drawn off continuously from the irradiated fluid flow B out of the tubular vessel 2. The fluid is drawn off at a rate controlled by a pump 24 and diluted with saline (before passing into the spectrophotometer 21. The flow rate of the fluid passing through the spectrophotometer 21 would typically be in the range 0.5 to 5.0 mL/min. After leaving the spectrophotometer, the fluid is run to waste 25. The spectrophotometer 21 is provided with a comparator 26 which continuously compares the increase in OD₃₁₄ of the irradiated fluid flow B above that of untreated fluid flow A, with predetermined upper and lower limits, and is coupled to an alarm device 27 so as to generate an alarm signal in response to excursions beyond these control limits. The comparator 26 may also be coupled to the main fluid flow rate controller 14 so as to adjust the fluid flow rate in respect to such changes, so as to maintain the OD₃₁₄ increase within acceptable operational limits.

### Example 1 - Monitoring UVC irradiation of Human Albumin

Samples of Human albumin (4.5% w/v aqueous solution at the pre-pasteurisation stage) were passed through a laboratory scale high efficiency static mixer UVC irradiation device (according to WO 00/20045) (obtained from Iatros Limited of Dundee, Scotland) at various different flow rates corresponding to different UV radiation dosages. Aliquots (2 ml) of the albumin solution before or after UV irradiation were collected and their absorbance studied using a double-beam ATI UNICAM UV/Vis Spectrometer UV2. The spectrometer used had two 1 cm path length cells, i.e. a sample cell and a reference cell. Two types of UV/Vis spectrum were recorded, namely a normal spectrum and a difference spectrum. The normal spectrum was obtained by scanning a sample placed in the sample cell against an empty reference cell, while during a difference spectrum-measurement, a non-UV treated protein sample (unless otherwise stated) was placed in the reference cell, so that the difference spectrum of UV and non-UV irradiated protein samples could be obtained. The scan range was 250 - 350 nm for most samples. 1-cm cell quartz cuvettes were used throughout the experiments as the sample and reference cells.

### Results

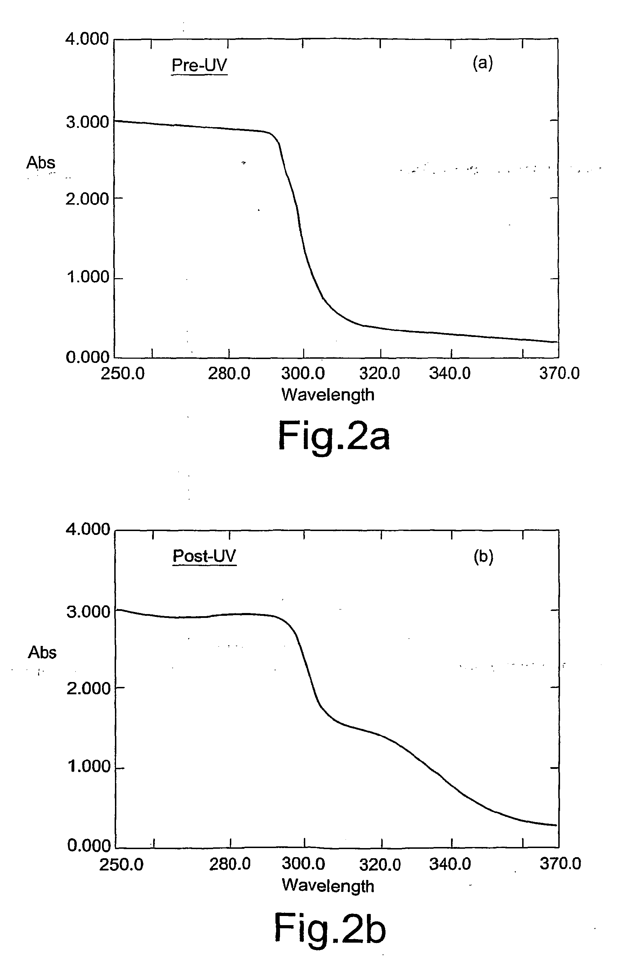

The absorption spectra of the non-UV and UV irradiated human albumin samples are shown in Figs. 2a and b. A "shoulder", increase in absorbance, appeared between 270 - 350 nm on the spectra for the UV irradiated samples. The height of the shoulder was related to UV dosage, the higher the UV dosage, the higher the shoulder.

### Example 2 - Monitoring increase in Absorbance of Human Albumin

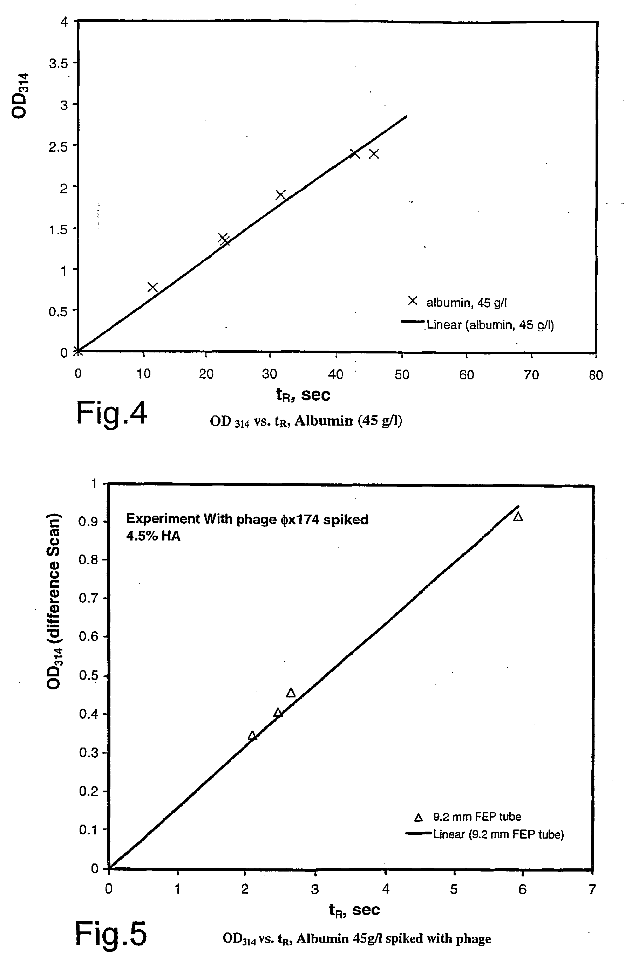

Substantially the same procedure as in Example 1 was carried out but in this case a single "difference" spectrograph was obtained by using a non-UV irradiated aliquot in the reference cell and a UV irradiated aliquot in the sample cell. The resulting spectrograph is shown in Fig. 3 which shows an absorbance peak extending across the wavelength range 305 to 325 nm, with an absorbance maximum at around 314 nm, i.e. OD₃₁₄. Fig. 4 shows the existence of a good correlation between OD₃₁₄ and the applied UV dosage expressed as the fluid residence time tᵣ. in the irradiation zone (which is given by the product of the length of the irradiation zone and the velocity of the fluid through the irradiation zone.

### Example 3 - Monitoring increase in OD₃₁₄ of Human Albumin spiked with Phage Øx174

The procedure of Example 2 was followed using Human Albumin (4.5% w/v aqueous solution) spiked with Phage Øx174 and a 9.2 mm internal diameter FEP tube. The phage φX174 was typically prepared with an initial titre of about 10¹⁰ plaque forming units/mL, and this was spiked into the test solution at ≤10%(v/v).
The OD₃₁₄ values obtained for a series of different UV radiation dosages are shown plotted in Fig. 5 which again shows a substantially linear relationship between OD₃₁₄ and UV radiation dosage.

### Example 4 - Monitoring increase in OD₃₁₄ of IgG

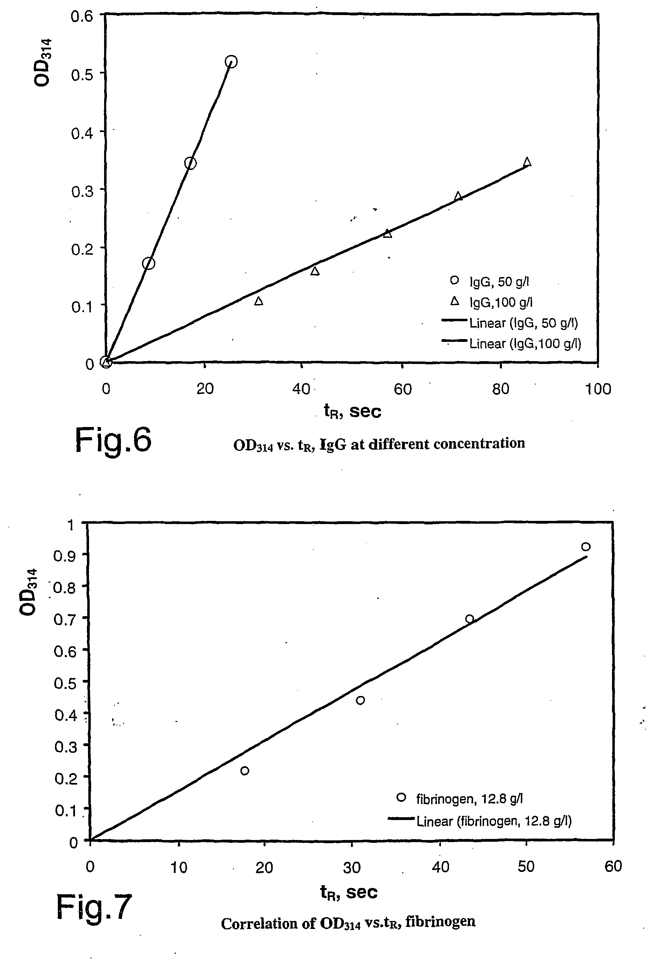

The procedure of Example 2 was followed using two different concentrations of IgG (50 and 100 g/l aqueous solutions). The absorbance OD₃₁₄ values obtained for a series of different UV radiation dosages are shown plotted in Fig. 6 which again shows a substantially linear relationship between OD₃₁₄ and UV radiation dosage.

### Example 5 - Monitoring increase in OD₃₁₄ of Fibrinogen

The procedure of Example 2 was followed using Fibrinogen (12.8 g/l aqueous solution). The absorbance OD₃₁₄ values obtained for a series of different UV radiation dosages are shown plotted in Fig. 7 which again shows a substantially linear relationship between OD₃₁₄ and UV radiation dosage at each of the two different concentrations.

### Example 6 - Monitoring increase in OD₃₁₄ of Human Albumin

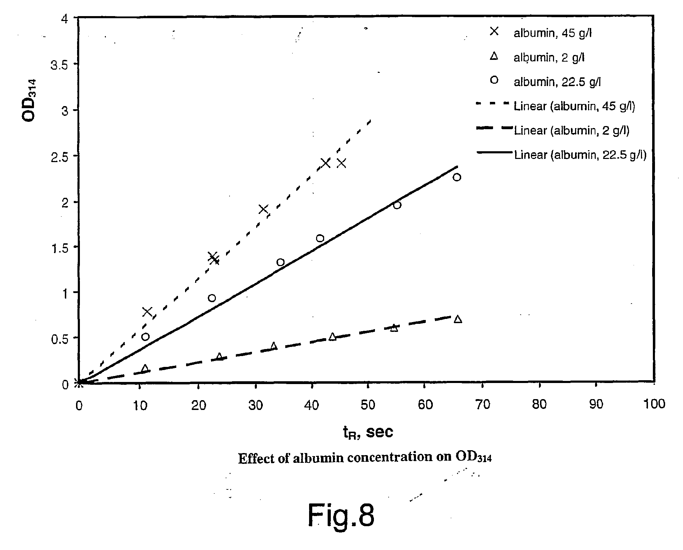

The procedure of Example 2 was followed using Human Albumin solution at a range of different concentrations (2.0, 22.5, and 45g/l aqueous solution). The absorbance OD₃₁₄ values obtained for a series of different UV radiation dosages are shown plotted in Fig. 8 which again shows a substantially linear relationship between OD₃₁₄ and UV radiation dosage for each of the different concentrations of human albumin solution.

## Claims

1. A method of monitoring the dosage of UV-irradiation received by a liquid containing at least one protein selected from albumin, immunoglobulins and fibrinogen; which method comprises the steps of UV-irradiating the liquid; and measuring the increase in absorbance which has a peak in the region from 305 to 325 nm, due to said UV-irradiation; so as to measure the dosage thereof.

2. A method according to claim 1, wherein the liquid also contains at least one micro-organism contaminant; and the step of UV-irradiating the liquid is carried out so that the liquid has received a UV-irradiation dosage sufficient to achieve a required level of inactivation of said at least one contaminant.

3. A method as claimed in any one of claims 1 and 2 which method is carried out on a liquid, which is substantially free of any dye or photosensitive chemical.

4. A method as claimed in any one of claims 1 to 3 in which method said increase in absorbance is measured for a stream of said liquid passing from a first position upstream of an UV irradiation zone to a second position downstream of said UV irradiation zone.

5. A method as claimed in any one of claims 1 to 4 which includes the preliminary step of introducing a said at least one protein into the liquid.

6. A method as claimed in any one of claims 1 to 5 in which said increase in absorbance is used to determine the UV irradiation received by a separate liquid exposed to said UV irradiation in series or in parallel with said liquid for which said increase in absorbance has been measured.

7. A method as claimed in claim 6 in which said liquid for which said increase in absorbance has been measured is passed through an UV irradiation zone both before and after said separate liquid is passed through said UV irradiation zone, and said increase in absorbance monitored or determined in both cases.

8. A method as claimed in any one of claims 1 to 7 wherein said increase in absorbance is measured continuously over a period of time.

9. A method according to any one of claims 1 to 8, which is carried out in an apparatus having a static flow mixing means with a multiplicity of mixer elements for repeatedly subjecting the liquid flow to a mixing operation comprising dividing and re-mixing of the liquid flow, which static flow mixing means extends along a flow path along at least an irradiation zone wherein the liquid is irradiated; and said apparatus includes liquid flow supply means formed and arranged for passing the liquid through said vessel, so that said liquid flow is subjected to at least 20 mixing operations.

10. A method as claimed in claim 9 wherein the apparatus comprises a single spectrophotometer device for measuring the increase in absorbency with the liquid upstream of the irradiation zone being used as a reference liquid in the spectrophotometer device.

11. A method as claimed in claim 10 wherein the apparatus comprises first and second spectrophotometers upstream and downstream of the irradiation zone and a comparator coupled thereto so as to obtain a difference in absorbance.

12. A method as claimed in claim 10, wherein the apparatus is provided with an alarm and/or liquid flow rate control device, and an output of said spectrophotometer is coupled to said liquid flow control device so as to adjust the liquid flow rate in response to excursions of the increase in absorbance outside predetermined limits, so as to generate an alarm signal, in response to excursions of the increase in absorbance outside predetermined limits, and/or bring said increase in absorbance back within said predetermined limits.

13. A method as claimed in claim 11, wherein the apparatus is provided with an alarm and/or a liquid flow rate control device, coupled to an output of said comparator, so as to generate an alarm signal, in response to excursions of the increase in absorbance outside predetermined limits, and/or bring said increase in absorbance back within said predetermined limits.

## Patentansprüche

1. Verfahren zum Überwachen der UV-Bestrahlungsdosis, die von einer Flüssigkeit empfangen wird, die wenigstens ein Protein enthält, das aus Albumin, Immunglobulinen und Fibrinogen gewählt ist; wobei das Verfahren die folgenden Schritte umfasst: UV-Bestrahlen der Flüssigkeit; und Messen der Erhöhung in der Absorption, die eine Spitze in dem Bereich von 305 bis 325 nm aufweist, als Folge der UV-Bestrahlung; um so die Dosis davon zu messen.

2. Verfahren nach Anspruch 1, wobei die Flüssigkeit wenigstens eine Mikroorganismus-Verunreinigung enthält; und der Schritt der UV-Bestrahlung der Flüssigkeit so ausgeführt wird, dass die Flüssigkeit eine UV-Bestrahlungsdosis empfangen hat, die ausreichend ist, um einen erforderlichen Grad einer Deaktivierung der wenigstens einen Verunreinigung zu erzielen.

3. Verfahren nach irgendeinen der Ansprüche 1 und 2, wobei das Verfahren auf einer Flüssigkeit ausgeführt wird, die im Wesentlichen frei von irgendeinem Farbstoff oder einem photoempfindlichen chemischen Stoff ist.

4. Verfahren nach irgendeinem der Ansprüche 1 bis 3, wobei in dem Verfahren die Erhöhung in der Absorption für einen Strom der Flüssigkeit gemessen wird, der von einer ersten Position stromaufwärts einer UV Bestrahlungszone an eine zweite Position stromabwärts von der UV Bestrahlungszone geht.

5. Verfahren nach irgendeinem der Ansprüche 1 bis 4, welches den vorläufigen Schritt zum Einführen des wenigstens einen Proteins in die Flüssigkeit hinein einschließt.

6. Verfahren nach irgendeinem der Ansprüche 1 bis 5, wobei die Erhöhung in der Absorption verwendet wird, um die UV Bestrahlung zu bestimmen, die durch eine getrennte Flüssigkeit empfangen wird, die der UV Bestrahlung seriell oder parallel mit der Flüssigkeit ausgesetzt ist, für die die Erhöhung in der Absorption gemessen worden ist.

7. Verfahren nach Anspruch 6, bei dem die Flüssigkeit, für die die Erhöhung in der Absorption gemessen worden ist, durch eine UV Bestrahlungszone geführt wird sowohl vor als auch nachdem die getrennte Flüssigkeit durch die UV Bestrahlungszone geführt wird, und die Erhöhung in der Absorption in beiden Fällen überwacht und bestimmt wird.

8. Verfahren nach irgendeinem der Ansprüche 1 bis 7, wobei die Erhöhung in der Absorption kontinuierlich über einer Zeitperiode gemessen wird.

9. Verfahren nach irgendeinem der Ansprüche 1 bis 8, welches in einer Vorrichtung ausgeführt wird, die einen statischen Flussmischmittel mit einer Vielzahl von Mischerelementen aufweist, um den Flüssigkeitsfluss wiederholt einer Mischoperation auszusetzen, die ein Aufteilen und Neumischen des Flüssigkeitsflusses umfasst, wobei sich der statische Flussmischmittel entlang des Flusspfads entlang wenigstens einer Bestrahlungszone erstreckt, in der die Flüssigkeit bestrahlt wird; und die Vorrichtung einen Flüssigkeitsfluss-Zuführungsmittel einschließt, die zum Führen der Flüssigkeit durch das Gefäß gebildet und angeordnet ist, so dass der Flüssigkeitsfluss wenigstens 20 Mischoperation ausgesetzt wird.

10. Verfahren nach Anspruch 9, wobei die Vorrichtung eine einzelne Spektrophotometereinrichtung zum Messen der Erhöhung des Absorptionsvermögens umfasst, wobei die Flüssigkeit stromaufwärts der Bestrahlungszone als eine Referenzflüssigkeit in der Spektrophotometereinrichtung verwendet wird.

11. Verfahren nach Anspruch 10, wobei die Vorrichtung einen ersten und einen zweiten Spektrophotometer stromaufwärts und stromabwärts der Bestrahlungszone und einen damit gekoppelten Vergleicher, um die Differenz in der Absorption zu ermitteln, umfasst.

12. Verfahren nach Anspruch 10, wobei die Vorrichtung mit einer Alarm- und/oder Flüssigkeitsflussraten-Steuereinrichtung versehen ist, und ein Ausgang des Spektrophotometers mit der Flüssigkeitsfluss-Steuereinrichtung gekoppelt ist, um so die Flüssigkeitsflussrate im Ansprechen auf Abweichungen der Erhöhung in der Absorption außerhalb vorbestimmter Grenzen einzustellen, um so ein Alarmsignal zu erzeugen, im Ansprechen auf Abweichungen in der Absorption außerhalb vorbestimmter Grenzen, und oder um die Erhöhung in der Absorption zurück in die vorbestimmten Grenzen zu bringen.

13. Verfahren nach Anspruch 11, wobei die Vorrichtung mit einer Alarm- und/oder Flüssigkeitsflussraten-Steuereinrichtung versehen ist, die mit einem Ausgang des Vergleichers verbunden ist, um so ein Alarmsignal zu erzeugen, im Absprechen auf Abweichungen der Erhöhung in der Absorption außerhalb vorbestimmter Grenzen, und/oder um die Erhöhung in der Absorption zurück in die vorbestimmten Grenzen zu bringen.

## Revendications

1. Méthode pour surveiller la dose d'irradiation UV reçue par un liquide contenant au moins une protéine choisie à partir d'albumine, d'immunoglobulines et de fibrinogène; laquelle méthode comprend les étapes d'irradier par rayons UV le liquide; et de mesure de l'augmentation d'absorbance qui a une pointe dans l'intervalle de 305 à 325 nm, due à l'irradiation UV; afin de mesurer sa dose.

2. Méthode selon la revendication 1, dans laquelle le liquide contient aussi au moins un contaminant, de type micro-organisme; et l'étape d'irradier par rayons UV le liquide est réalisée de manière à ce que le liquide reçoive une dose d'irradiation UV suffisante pour atteindre un niveau requis d'inactivation dudit au moins un contaminant.

3. Méthode comme revendiquée dans l'une quelconque des revendications 1 et 2 qui est réalisée sur un liquide, lequel est substantiellement libre de tout colorant ou produit chimique photosensible.

4. Méthode comme revendiquée dans l'une quelconque des revendications 1 à 3 dans laquelle l'augmentation d'absorbance est mesurée pour un courant du liquide passant d'une première position en amont d'une zone d'irradiation UV à une seconde position en aval de la zone d'irradiation UV.

5. Méthode comme revendiquée dans l'une quelconque des revendications 1 à 4 qui comprend l'étape préliminaire d'introduire ladite au moins une protéine dans le liquide.

6. Méthode comme revendiquée dans l'une quelconque des revendications 1 à 5 dans laquelle l'augmentation d'absorbance est utilisée pour déterminer l'irradiation UV reçue par un liquide séparé exposé à l'irradiation UV, en série ou en parallèle avec le liquide pour lequel l'augmentation d'absorbance a été mesurée.

7. Méthode comme revendiquée dans la revendication 6 dans laquelle le liquide pour lequel l'augmentation d'absorbance a été mesurée est passé au travers d'une zone d'irradiation UV à la fois avant et après que le liquide séparé est passé au travers de la zone d'irradiation UV, et l'augmentation d'absorbance est surveillée ou déterminée dans les deux cas.

8. Méthode comme revendiquée dans l'une quelconque des revendications 1 à 7 dans laquelle l'augmentation d'absorbance est mesurée de façon continue sur une période de temps.

9. Méthode selon l'une quelconque des revendications 1 à 8, qui est réalisée dans un appareillage qui a un moyen statique de mélange de flux, avec une multiplicité d'éléments mélangeurs pour soumettre de façon répétée le flux liquide à une opération de mélange comprenant la séparation et le re-mélange du flux liquide, lequel moyen statique de mélange de flux s'étend le long d'un chemin d'écoulement le long d'au moins une zone d'irradiation dans laquelle le liquide est irradié; et l'appareillage comprend un moyen d'approvisionnement du flux liquide formé et disposé pour passer le liquide au travers d'un récipient, de telle façon que le flux liquide est soumis à au moins 20 opérations de mélange.

10. Méthode comme revendiquée dans la revendication 9 dans laquelle l'appareillage comprend un unique dispositif spectrophotomètre pour mesurer l'augmentation de la capacité d'absorbance, le liquide en amont de la zone d'irradiation étant utilisé comme liquide de référence dans le dispositif spectrophotomètre

11. Méthode comme revendiquée dans la revendication 10 dans laquelle l'appareillage comprend un premier et un second spectrophotomètre en amont et en aval de la zone d'irradiation et un comparateur qui leur est couplé de façon à obtenir une différence d'absorbance.

12. Méthode comme revendiquée dans la revendication 10 dans laquelle l'appareillage est muni d'une alarme et/ou d'un dispositif de contrôle du débit du flux liquide, et une sortie du spectrophotomètre est couplée avec l'équipement de contrôle du flux liquide de façon à régler le débit du flux liquide en réponse aux dépassements de l'augmentation d'absorbance en dehors de limites prédéterminées, de manière à générer un signal d'alarme en réponse aux dépassements de l'augmentation d'absorbance en dehors de limites prédéterminées, et/ou de ramener l'augmentation d'absorbance dans les limites prédéterminées.

13. Méthode comme revendiquée dans la revendication 11, dans laquelle l'appareillage est muni d'une alarme et/ou d'un équipement de contrôle du débit du flux liquide, couplé à une sortie du comparateur, de façon à produire un signal d'alarme en réponse aux dépassements de l'augmentation d'absorbance en dehors de limites prédéterminées et/ou de ramener l'augmentation d'absorbance dans les limites prédéterminées.
